(19) Europäisches Patentamt
European Patent Office
Office européen des brevets

(11) Publication number: 0 367 432 A1

# (12) EUROPEAN PATENT APPLICATION

(21) Application number: 89310496.8

(51) Int. Cl.5: C12M 1/40 , G01N 27/12

(22) Date of filing: 12.10.89

(30) Priority: 31.10.88 GB 8825437

(43) Date of publication of application:
09.05.90 Bulletin 90/19

(84) Designated Contracting States:
DE FR IT NL

(71) Applicant: PLESSEY OVERSEAS LIMITED
Vicarage Lane
Ilford Essex IG1 4AQ(GB)

(72) Inventor: Sethi, Rajinder Singh
68 Westone Avenue
Northampton, NN3 3JG(GB)
Inventor: Gray Stephens, Lauren Diana
9 Kingfisher Close
Bourn Cambridgeshire, CB3 7TJ(GB)
Inventor: Bruce, Neil Charles
93 Norwich Street
Cambridge, CB2 1ND(GB)
Inventor: Lowe, Christopher Robin
The Limes Hempstead
Saffron Waldon Essex, CB10 2PW(GB)

(74) Representative: Pritchard, Evan
Intellectual Property Department The
Plessey Company plc Vicarage Lane
Ilford Essex IG1 4AQ(GB)

## (54) Biosensor device.

(57) A biosensor device comprising an electrical insulation layer 11 carried on a substrate 1 of semiconductor material, the layer supporting a pair of substantially parallel thin film electrical conductors 12, electrical connection means for said conductors, a space between said conductors defining a reaction cell such that an electrical or thermal characteristic of a reaction component 13 therein can be detected at said connection means, in which the semiconductor substrate material in the cell region has a surface structure including a roughly textured area where an enlargement of the substrate surface is present. The enlarged surface area appears to promote bonding of biological material in the cell and thus it enhances the electrical signal output.

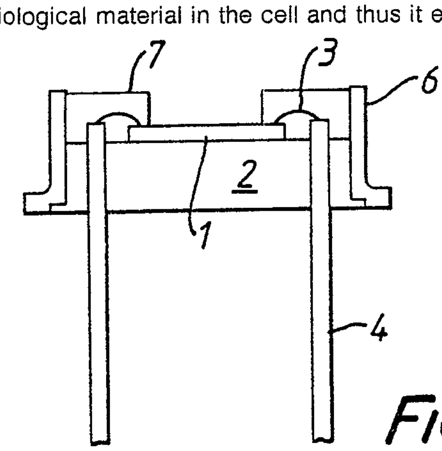


FIG. 1.

EP 0 367 432 A1

Xerox Copy Centre

# BIOSENSOR DEVICE

This invention relates to a biosensor device. It relates to a device which can be considered as a modification of that disclosed in patent application No. 2204408, dated 4th March 1987, although the present invention is not of course restricted to biosensors of that particular type.

The aforementioned patent application relates to a biosensor device comprising an electrical insulation layer carried on a substrate of semiconductor material, the layer supporting a pair of substantially parallel thin film electrical conductors, electrical connection means for said conductors, a space between said conductors defining a reaction cell such that a reaction component when present in said cell will bridge said conductors permitting an electrical or thermal characteristic of the component to be detected at said connection means.

It has now been discovered that a modification can be made in the device construction which will allow a greater sensitivity to be obtained in making the electrical measurement.

According to the present invention, there is provided a biosensor device having electrical conductors separated by a support surface which is in contact with a biological sample when this is present in the cell, in which the support surface has a surface structure including an enlargement of the area of the surface material which can be brought into contact with said sample.

The support surface may be of a semiconductor material, such as silicon. The semiconductor substrate may be provided with an electrically insulating surface film.

According to a feature of the invention, there is provided a biosensor device comprising an electrical insulation layer carried on a substrate of semiconductor material, the layer supporting a pair of substantially parallel thin film electrical conductors, electrical connection means for said conductors, a space between said conductors defining a reaction cell such that an electrical or thermal characteristic of a reaction component therein can be detected at said connection means, in which the semiconductor substrate material in the cell region has a surface structure including a roughly textured area where an enlargement of the substrate surface is present.

In one embodiment, there is provided a method of constructing a biosensor device, the method comprising the steps of taking a wafer of semiconductor material, selecting one major surface as a potential active wafer region, etching said surface so as to enlarge the surface area, oxidising said surface to form an electrical insulation film, depositing a pair of substantially parallel electrical conductors on said film and providing electrical connection means for the conductors.

In a further embodiment, there is provided a method of constructing a biosensor device, the method comprising the steps of taking a wafer of semiconductor material, selecting one major surface as a potential active wafer region, oxidising said surface to form an electrical insulation film, depositing a metal layer on the oxide film, etching said metal layer so as to enlarge the surface area, depositing a pair of substantially parallel electrical conductors on said layer and providing electrical connection means for the conductors.

The major surface of the semiconductor material may be a rear side of the wafer,as hereinafter defined. The etching step may be effected by an electrochemical process.

By way of example, some particular embodiments of the invention will now be described with reference to the accompanying drawings, in which:-

Figure 1 is a diagrammatic cross-section of a biosensor device according to the invention,

Figure 2 is a plan view of a part of the device, showing the arrangement of electrical conductors,

Figure 3 is a diagrammatic cross-section of part of the sensor device, in use,

Figure 4 is an electrical circuit diagram of the component arrangement required to make the necessary electrical property measurements,

Figure 5 is a graph of a calibration curve for the electrical circuit,

Figures 6 to 13 are electron micrographs of areas of a silicon semiconductor surface which have been given particular surface treatments,

Figure 14 is a graph illustrating the conductance output for biosensor devices having differing surface textures,

Figure 15 is a graph showing the response with respect to time for a rough surface biosensor device, and,

Figure 16 is a graph showing the conductance measurements for an immobilised urea/urease system against varying concentrations of urea.

This invention relates to the modification of the surface of a biosensor device which can be utilised in a multi-analyte application and which is based on a surface conductance measurement technique.

The basic device can comprise a pair of substantially parallel electrical conductors supported on a

surface of electrical insulation material. By the words 'substantially parallel' as used in this specification, it is meant to include arrangements such as a single electrode which may be folded or serpentined to give a parallel arrangement of parts of this conductor. In addition, the expression includes an arrangement of two separate electrodes which could have interdigitated parts.

With the conductors supported on the surface, an analyte reagent mixture may be placed thereon thus enabling an electrical property of the analyte reagent mixture to be measured and changes in that property to be determined.

In a preferred embodiment of the biosensor device, the conductors are laid upon the surface of a silicon chip. This operation may be effected simultaneously with the fabrication, on the same chip, of ancillary circuitry to enable the electrical property measurements or changes to be determined. Such an arrangement permits very small parameters or parameter changes to be measured without the influence of noise, the presence of which can be inherent when external electrical circuitry is employed. Further, the device may include a quantity of an immobilised reagent material which is placed so as to overlie the conductors. Such a reagent material may be an enzyme which alters the conductance of an applied analyte.

The reagent material may form part of an enzyme/analyte system applied to the sensor to enable measurements to be made.

Advantageously, a further pair of conductors is fabricated in the same way and preferably at the same time, to act, in use, as a control means whereby the effects of temperature and other variations may be eliminated.

The fabrication of the basic device of the invention involves the following main operations on a silicon wafer: thermal oxidation; metallisation; photolithography and etching; sawing; chip bonding; wire bonding and encapsulation; and deposition of the required enzyme/immobilisation material. Some of these operations make use of existing semiconductor device fabrication techniques. The details are given below:

(1) A silicon wafer is thermally oxidised in a furnace using a standard oxidation process to provide a film approximately 550 nanometres in thickness of silicon dioxide ($SiO_2$) on its surface.

(2) A multimetal coating is next deposited onto the oxidised silicon surface using a standard sputter deposition process, electron-beam evaporation or thermal evaporation under vacuum. Four alternative examples of metallisation scheme employed are: titanium/gold, titanium/platinum/gold (Ti/Pt/Au), chromium/gold and chromium/platinum/gold. The thickness of each of the metals titanium, platinum and chromium was usually around 100 nanometres whereas that of gold varied between 1,000 nanometres to 3,300 nanometres depending upon the particular application.

(3) The required substantially parallel metal conductor tracks/patterns 1,200 nanometres to 3,500 nanometres thick and 5,000 nanometres wide are produced by using the following photolithographic process(es) and metal etching.

<u>Process (a): (Resist material thickness about 500 nanometres)</u>

A small quantity of 3-mercapto-propyl-trimethoxy-silane liquid (MPTs: sold by Fluke AG, Switzerland) was spread on the metallised wafer and it was then spun on a motor-driven turntable at a rate of 5,000 rpm for forty seconds. The resulting layer which was formed acted as a surface adhesion promoter. A layer of a positive photolithographic resist material AZ4040 (Hoechst, West Germany) was next spread on the wafer, it was spun at 3,000 rpm for sixty seconds and then prebaked in an oven at a temperature of 90 °C for two minutes. The wafer was aligned with the mask (one repeat pattern of which is depicted in Figure 2) on a MIB-21 model mask aligner machine (Karl Suss, West Germany) and exposed to an ultraviolet radiation dose of 72 millijoules per square centimetre. Photographic development took place in K400-AZ developer (Hoechst) (at a concentration of 1:4 in dilution with water) for forty seconds and the wafer was then postbaked at 90 °C for two minutes.

<u>Process (b): (Resist material thickness about 3,500 nanometres)</u>

A small quantity of the surface adhesion promoter MPTS liquid was spread on the metallised wafer and spun at 4,000 rpm for sixty seconds. Next, a layer of a positive resist material AZ4330 (Hoechst) was spread on the wafer, it was spun at 3,000 rpm for sixty seconds and then prebaked at 90 °C for five minutes. The wafer was aligned, as under process (a), exposed to an ultraviolet radiation dose of 225 millijoules per square centimetre, developed in a photographic developer K400-AZ (Hoechst) (at a concentration of 1:3 in dilution with water) for sixty seconds and then postbaked at 90 °C for fifteen minutes. It

was then further heated at 110° C for five minutes to flow bake the resist. The latter process results in a rounding of the edges of the resist patterns which subsequently helps in producing an evenly etched metal pattern. The required metal patterns were produced by etching metals through developed resist patterns either by dry etching techniques such as ion-beam milling for removing all the metals (gold, platinum, titanium and chromium) or by a combination of standard wet etching (of gold, titanium and chromium) and dry etching (of platinum) techniques. It is preferred to employ resist deposition process (a) for wet etching whilst process (b) is preferred for dry etching.

(4) The remaining resist material was subsequently removed by dissolving in acetone or standard AZ resist remover (Hoechst). The silicon wafer was finally subjected to a washing sequence involving deionised water, acetone, methanol and isopropyl alcohol. The wafer was then sawed by a standard process to produce discrete silicon chips each one containing one to three surface devices.

(5) As depicted in Figure 1, the chip was mounted on and bonded to a standard twelve pin TO5 header package 2. The bonding was effected using an epoxy resin bonding material or in a different embodiment a gold tin (80:20) eutectic solder composition. When solder is employed for bonding, it is preferable to metallise the bonding surface of the silicon chip since this improves the adhesion of the chip to the header. The connections to the devices were made by a thermocompression wire bonding technique (using lengths of gold wire 3 of thickness about 25,000 nanometres) secured onto the device pads and pin-heads of the pins 4 of the package 2. An open-ended can 6 was welded to the header. The electrical connections to the devices were then encapsulated in a body of epoxy resin 7 leaving exposed an appropriate number of the parallel conductor networks.

Figure 2 shows on a greatly enlarged scale the microcircuit surface. In this embodiment, the discrete silicon chip 1 carried three surface devices 8 each of which comprised a pair of substantially parallel electrical conductors. It will be seen that each device 8 is formed of two long serpentined electrical conductors the two ends of each conductor terminating in a contact pad 9 located on the outer edge of the chip 1. The two conductors of each device 8 are serpentined together so as to provide a portion of each conductor which is in a substantially parallel relationship with a similar portion of the other conductor.

It will be clear that in a different embodiment it will be possible to position the electrical conductors in some alternative manner which will still give the required parallel relationship between parts of the conductor(s). One such alternative construction would be to have an interdigitated arrangement. A single electrical conductor which was laid down in a serpentine arrangement could also be used since the necessary pair of parallel conductors could be formed from different parts of the same metal strip.

Figure 3 shows in a further enlargement a cross-sectional view of part of the chip 1 supporting the electrical conductor areas.

In this view, the silicon substrate material constituted by the chip 1 has had a surface layer 11 of silicon dioxide formed on its upper surface. The surface layer 11 is of course an electrical insulation material and this serves to support the metal electrical conductor areas 12. The view in cross-section shows two conductor areas 12 and a volume of an immobilised enzyme material 13 has been placed between these two areas. The area of electrodes and enzyme material 13 is then covered over with a quantity of an analyte 14.

The miniaturised surface conductance device offers potential for the measurement of many biological substrates when their complementary enzymes are immobilised across the surface of the electrical conductor networks. Enzymes are chosen which by their specific catalytic action generate or consume ionic species and thereby lead to a change in solution conductance. The procedure is generally applicable to deaminases, amidases, oxidases, proteases, nucleases, other hydrolases, decarboxylases, kinases, lipases, phosphatases and many other enzymes.

Urea Biosensor

For example, the enzyme urease catalytically cleaves a neutral substrate urea into positively charged ammonium ions and negatively charged bicarbonate and hydroxyl ions:

$$(NH_2)_2CO + 3H_2O \xrightarrow{\text{urease}} 2NH_4^+ + HCO_3^- + OH^-$$

Thus, urease immobilised to the surface of the parallel conductor pattern would enable the presence of urea in an analyte to be detected by observing conductance changes measured across the two tracks of the

above pattern (Figure 3) as the enzyme cleaves the urea.

Construction and Operation

The electrical circuit about to be described was designed to make use of conventional alternating current conductimetric monitoring techniques in order to reduce Faradaic processes, double-layer charging and concentration polarisation at the microelectrode surface.

The conductance biosensor was normally operated in a dual cell configuration comprising a reference pair and a sample pair of the parallel electrical conductors. Accordingly, therefore, the circuit diagram as depicted in Figure 4 shows connections for a sample conductance cell 16 which receives a power supply from a monolithic integrated circuit waveform generator 17 (Model No. RS8039CC from RS Components Ltd., Corby, Northants, UK). An output terminal of the cell 16 is connected to a high input impedance FET operational amplifier 18 (RS Components, CA3140) and this has a fixed feedback resistor 19 (ten megohms plus or minus 1%) with the conductance cell forming an input resistor.

An output signal from the amplifier is taken to an RMS-DC converter integrated circuit 21 (RS Components, AD563A) such that the output voltage could be monitored on a chart recorder (Gould BS272) and or a digital multimeter (Hewlett Packard 3468A).

The circuit as just described would be suitable for operation in single cell mode with the output voltage from the RMS-DC converter circuit 21 being taken directly to the chart recorder. As already mentioned, however, a dual cell configuration is preferred and thus the circuit of Figure 4 includes the sample conductance cell 16 together with a reference cell 22. The full circuit uses two identical sine wave generator 17 inverting operational amplifier 18 units with the outputs from the two reference and sample RMS-DC converters 21 being taken to a conventional differential amplifier 23 and thence to a chart recorder/digital multimeter output terminal 24.

The biosensor cells (as mounted on a T05 header package) were temperature equilibrated to 30° ± 0.1 °C by means of a glass water jacket connected to a Julabo 20B heating/circulating water bath. If the biosensor chip is mounted on a ceramic chip carrier, it is heated by a suitable Peltier block heater/cooler placed directly in intimate contact underneath the carrier and this is fitted in a specially made snap-on edge contact socket holder contained in a thermally insulated box. The socket holder is connected to the miniature BNC sockets which are fitted on the outside of the box for making external coaxial connections. The temperature of the chip is controlled at 30° ± 0.1 °C with the help of a thermistor fitted close to the chip and an external temperature control unit. About four minutes temperature equilibration of the sensor was allowed before taking the measurements.

In operation of the circuit of Figure 4, the integrated circuit waveform generators 17 apply a low distortion sine wave of frequency 1kHz and amplitude zero volts plus or minus 5mV to the sample and reference cells. The inverting operational amplifiers 18 provide gain and serve to amplify the responses from the cells. The output signals from the amplifiers 18 are converted into DC voltages via the integrated circuit RMS-DC converters 21.

The instrument was calibrated by relating the DC output of the RMS-DC converter 21 to reciprocal ohms (Siemens) by connecting low temperature coefficient 1% tolerance, 0.25W metal-film type resistors (having values between 56K and 10 megohms) across the sample pair of microelectrodes in order to simulate solution conductance changes. This calibration procedure permitted the output voltage of the conductance biosensor to be related to the S.I. units (Siemens) measurement for conductance.

Figure 5 is a graph of a calibration curve for the electrical circuit where the horizontal axis shows conductance (in microSiemens) against output voltage (in volts) on the vertical axis. The output voltage from the RMS-DC converter 21 is seen to be linearly related to the calibration conductance with a correlation coefficient of 0.998 as deduced by linear regression analysis. This calibration curve suggests that the output voltage from the RMS-DC converter 21 will be linearly related to the change in ionic strength generated during the initial period of an enzyme-catalysed reaction, provided that the basic ion strength of the buffered solution is low enough to permit an adequate signal to background noise ratio. For this reason, a buffer of low intrinsic conductance, imidazole, was used throughout for the measurements.

Enzyme Immobilisation

A urease (urea amidohydrolase, EC 3.5.1.5) gel was made by carefully adding 10 microlitres of a mixture of equal volumes of enzyme (100 mg/ml), bovine serum albumin (100mg/ml) and glutaraldehyde

(2.5% by volume) solution, all made up in a 5mM imidazole-HCl buffer, pH 7.5 solution. An enzyme albumin gel formed after nine or ten minutes at 20 °C and it was then washed exhaustively with the 5mM imidazole-HCl buffer, pH 7.5 solution.

This pre-gelled urease mixture was introduced to the microelectric pattern on the chip with a hand-held capillary tube. This immobilisation technique was found to allow the introduction of different enzyme systems at each of the microelectrode patterns on the same chip, that is this makes it possible to produce a multi-enzyme biosensor on the same chip.

Sample Measurement Protocol

The microelectronic conductance biosensor was initially calibrated against known concentrations of urea as explained in the aforementioned patent application. Imidazole-HCl buffer (2mM, pH 7.5, 180 microlitres) was added to the device equilibrated to 30 °C, whence, once a stable baseline had been achieved after a time period of from thirty to sixty seconds, a known concentration of urea (0-100mM) (20 microlitres) was added. The output voltage from the differential amplifier 23 was monitored over a three to four minute time period to ensure linearity.

The determination of the electrical conductivity of solutions depends on the accurate measurement of the electrical resistance between two electrodes of defined geometry immersed in the conducting solution. In the differential microelectronics conductance biosensor described here, the device comprises identical pairs of multimetal electrodes fabricated on a silicon substrate in a planar configuration so that these requirements can be achieved.

The present invention relates to a modification of the surface of silicon by various chemical and electrochemical treatments which can result in an overall enhancement of the bioelectrical signals obtained from the biosensor device. This treatment can result in a useful improvement in the sensor signal for a urea/urease system using treated silicon as compared with standard polished silicon.

In the aforementioned patent application, all the processing to produce the silicon device had been carried out on the normal polished 'smooth' side or what is called the front side of the silicon wafer. In the new process, the surface of the device is modified by either pretreating the single crystal silicon surface prior to a thermal oxidation stage or by post-treating it after a metallisation stage during the fabrication of the device.

For clarity in the present specification, it is to be understood that the silicon wafer as it is received from the manufacturer has two differently textured sides. The side which is normally used for producing semiconductor devices has a smooth surface which has been produced by a polishing operation and this is sometimes called the 'front' side of the wafer. The second side of the wafer is not normally used for semiconductor devices and this is therefore left in an unfinished condition after slicing the drawn silicon boule. This second side is called the back, the rough or the rear side of the wafer. In this specification, the terms 'front side' and 'rear side' will be used in referring to the two original sides of the wafer.

The present application discloses that it is not essential to use the front side of the wafer for biosensor construction. Whilst the front side, possibly with suitable surface treatment, can enable an excellent biosensor to be constructed, an unexpected benefit can occur if the sensor is formed on the wafer rear side. Of course, the wafer front side might still be needed for ancillary devices associated with the working of the biosensor. These devices might include a transistor amplifier, and the thermistor for controlling the temperature of the biosensor chip.

Pretreatment of the silicon surface is carried out in one of three different ways:

a) cleaning and etching treatment of the front or rear side of the single crystal silicon wafer, n- or p-type,

b) a deposition of polysilicon on the rear side or on the lightly etched front side of the wafer by standard technique, or

c) a deposition of polysilicon on the front side of the wafer by standard techniques. The cleaning and etching of the silicon surface may be carried out by wet chemical reagents such as alkalis and acids, or by dry etching techniques such as sputtering or ion-beam milling.

Post-treatment of the, metallised oxidised silicon surface is also carried out in one of three different ways:

a) by standard wet chemical etching reagents to suitably 'roughen' the metallised surface,

b) by dry etching using an argon ion or helium ion sputtering technique, or,

c) by a suitable electrochemical treatment given to the assembled device or to the wafer prior to the sawing stage in the fabrication of the device.

The preferred process employed the chemically pretreated rear side of the p-type (resistivity: 3.75-6.25ohm.cm$^{-1}$) boron doped <100> silicon wafer followed by the electrochemical post-treatment of the assembled device. The wafers were produced by the Czochralski method and were supplied by the Monsanto Company (U.S.A.) and had oxygen concentration between 26-35 ppma and carbon about 0.1ppma present as precipitate in the rear side of the wafer for intrinsic gettering. The polished n-type (resistivity: 1.2-2.8 ohm.cm1) phosphorus doped <100> silicon wafers produced by the Czochralski method which were supplied by Wacker-Chemitronic (West Germany) also produced good results.

Pretreatments - cleaning and etching of silicon wafer material

A clean silicon surface by definition is one free of contaminants, such as residues of mounting wax, lapping compounds or traces of solvents. An initial check for cleanliness in usually made by visual inspection under a bright fluorescent light. In general, cleaning procedures not only remove dust and dirt but they also have been found to change the surface characteristics of the wafers drastically. Features such as roughness, surface topography and work damage may have a considerable influence on the various device characteristics. Some examples of cleaning procedures that may be chosen according to the requirements of the device process and the results of some typical surface preparations are demonstrated by scanning electron microscope photographs which have been reproduced in the accompanying Figures 6 to 13.

1. Ultrasonic Cleaning

If no change in the surface topography of 'as cut' and lapped wafers is required then the wafer material can be immersed in an ultrasonically agitated cleaning solvent. Impurities, if present, are removed from the wafer surface by this method and the resulting etching effect can be absolutely negligible, that is roughness and surface topography are not changed. Ultrasonic cleaning is often employed for applications where the same surface characteristics as those appearing after slicing or lapping are required. Allowances must be made for the presence of local differences in ultrasonic energy as these can cause variations in the degree of attack on the silicon wafer surface.

Examples

Figure 6

(scale, one cm = 16 micrometres)
Front side (n- or p-type).
Ultrasonically cleaned.

Figure 7

(scale, one cm = 16 micrometres)
Oxidised front side (n- or p-type)
Shows no change after silicon dioxide formation (550 nanometres in thickness)

Similarly, when the oxidised wafer material is further coated with Ti/Pt/Au, this metallisation layer having thicknesses as already described, it shows no change in surface topography under microscopic examination.

2. Chemical Cleaning

Chemical cleaning of the silicon wafer material with dilute alkali solution for a very short period of time was employed where the fabrication process involved wet etching with acid. This surface treatment only removes less than 0.5 micrometres of silicon from the wafer surface. It is recommended by one particular silicon wafer supplier (Wacker, in Wacker Technical Bulletin No. 01.86) that the chemical attack of an acid

etch can be more uniform if the starting silicon material has been subjected to a prior chemical cleaning process.

3. Alkali Etching

To remove work damage from the lapped rear side of the silicon wafer, a strong alkali solution was employed to remove ten micrometres or more of the material. The surface topography of the silicon wafer is very different when it is compared with that of an ultrasonically cleaned front side (of Figures 6 and 8).

Examples:

Figure 8

(scale, one cm = 25 micrometres)
Rear side of n-type silicon wafer, etched with strong alkali.

Figure 9

(scale, one cm = 25 micrometres)
Oxidised (Figure 8) silicon surface.
(oxide film thickness = 550 nanometres)
There is no change in surface topography after oxide formation.

Similarly, when the oxidised wafer material is further coated with Ti/Pt/Au (with metal thicknesses as already described) there is no change in its surface topography when observed under microscopic examination.

4. Acid Etching

Standard acid etching was also employed to remove work damage from the lapped rear side of the silicon wafer to produce a lightly structured surface. This surface is relatively smooth (Figure 10) as compared to that etched with an alkali (Figure 8)

Examples

Figure 10

(scale, one cm = 25 micrometres)
Rear side of p-type silicon wafer, etched with acid.

Figure 11

(scale, one cm = 25 micrometres)
Oxidised (Figure 10) silicon surface (oxide film thickness = 550 nanometres).
There is no change in surface topography after oxide formation.

Similarly, when the oxidised wafer material is further coated with standard Ti/Pt/Au, there is no change observed in its surface topography under microscopic examination.

Post-treatment

1. Etching of Gold Film

Metallised front oxidised silicon wafers (n- or p-type) were subjected to treatments such as ion beam milling, sputter etching and wet chemical etching the object of which was mainly aimed at roughening of and or causing damage to the metal surface. Of these techniques, only wet chemical etching using standard potassium iodide-iodine ($KI-I_2$) etchant has produced encouraging results. Some typical microscopic pictures of treated surfaces are shown below.

Figure 12

(scale, one cm = 10 micrometres)
Sputtered deposited coating of Ti.Pt/Au on oxidised front side (n- or p- type).
Ion-beam milling or sputter etching at low energies produced only a small change in surface topography of the gold film on the above sample. Since these processes are more usually carried out at high energies they are likely to produce some surface roughening and/or damage.

Figure 13

(scale, one cm = 0.4 micrometres)
Wet etched (Figure 12) surface using standard $KI-I_2$ etchant. This appears to match the rear side of p-type acid etched silicon wafer material (Figure 10), except that the grain size or degree of roughness is on a smaller scale.

2. Electrochemical Treatment

The post-treatment of the surface of the assembled device was performed by covering the device pattern with a small quantity of a buffer solution of potassium phosphate (at a concentration of 100mM, pH = 7.0) and electrochemically cycling the device at $50mV.sec^{-1}$ between zero and + 1V using a platinum wire counter electrode, prior to enzyme deposition. A potentiostat controlled by a microcomputer via a Cambridge Electronic Design Model 1401 Interface was employed for ramping the applied potential and the temperature of the device was controlled at 30° ± 0.1 °C during this operation. A period of up to fifteen minutes cycling produced an increase of about three times in the apparent conductance signal for a 1mM urea/urease model system using a device made from p-type rear side silicon. This represents an overall enhancement of about twelve times in the electrical signal output as compared with that from a control p-type front side silicon device. However, the prolonged cycling for a period of sixty minutes resulted in a decrease of the signal by about forty percent. The step of poising the sensor device at +0.4V and -0.4V against the platinum counter electrode was also found to produce lower electrical signals for the urea/urease system.

For a comparison of the results obtained with the various treatment processes, the following terminology will be used:

1) Front side device

These are p-type front side devices as disclosed in the aforementioned patent application. The results of this test can therefore be considered as a 'control' result.

2) Rear side device

There is a modified device based on using the p-type rear side (acid treated) silicon or the rear side of the silicon wafer material.

3) Rear side and electrochemical treatment device

There is a further modified device based on p-type rear side (acid treated) silicon wafers, plus a post-treatment electrochemical process for fifteen minutes as described earlier.

An initial assessment of the devices produced by different surface treated silicon wafers was carried out

by comparing the conductance signals produced by using standard potassium chloride (KCl) solutions of varying concentrations in the range 10mM to 3M. For this purpose KCl solutions were prepared in a 'Super Q' quality of water (Millipore: 18 megohm grade).

Solution volumes of twohundred microlitres were introduced into the microelectronic conductance device and equilibrated to a temperature of 30° ± 0.1°C for about four minutes. The conductance output from each of the three microelectrode pairs, immersed in varying concentrations of KCl solutions was recorded. An average conductance output was obtained from three separate volumes of KCl solutions. This was repeated for both front side and rear side silicon devices. The results are plotted in Figure 14.

In the graph of Figure 14, the vertical axis measures output conductance, in volts, of the device. The horizontal axis gives potassium chloride concentration in moles. The conductance output for the front side surface is shown by the dotted line, whilst that from the rear side surface is shown by the solid line.

The expected increase in conductance with increasing concentrations of KCl was observed. At about 1.5M, the maximum signal for a device formed from a front side surface was about 950mV. This signal was increased to about 1500mV for devices with a rear side surface due to the increased surface area present in the device.

Immobilised Urease-Urea System

A quantity of urease (from Jack Bean, Sigma Chemical Company) was formed into a gel matrix as explained earlier by forming a cross-linked enzyme-albumin membrane with glutaraldehyde. The gel was next introduced to the metal network on the chip and varying concentrations of urea in the range 1 mM to 100mM made in the imidazole buffer (2mM, pH = 7.5) were added to the urease gel.

The results obtained for 1 mM urea for variously treated silicon surfaces is shown in Table 1. The results obtained for electrochemically post-treated assembled devices utilising a p-type rear side surface are given in Table 2.

TABLE 1

| S. No. | Surface treatment | | Steady-state output voltage signal, mV |
|---|---|---|---|
| 1. | "Front side p-type": | p-type front side silicon surface. | 21 |
| 2. | "Front side n-type": | n-type front side silicon surface | 70 |
| 3. | "Front side polysilicon": | polysilicon deposited onto p-type front side silicon. | 33 |
| 4. | "Rear side p-type": | p-type rear side (acid treated) silicon surface. | 84 |

TABLE 2

| Working Electrode: Microconductimetric sensor | | |
|---|---|---|
| Counter Electrode: Platinum wire | | |
| Electrolyte: Potassium hydrogen phosphate (100mM, pH = 7.0) | | |
| S. No. | Electrochemical Post-treatments of "Rear side p-type" sensor devices. | Steady-state output voltage signal. mV |
| 1. | Sensor cycled between OV and + 1.0V at 50mV $sec^{-1}$ | 254 |
| 2. | Sensor poised at +0.4V | 16 |
| 3. | Sensor poised at -0.4V | 54 |
| 4. | Sensor poised at 0V | 56 |
| 5. | "Rear side p-type" sensor device. | 84 |

A typical response of rear side silicon devices electrochemically cycled for fifteen minutes to a 2mM urea/urease system is shown in Figure 15.

In the graph of Figure 15, the vertical axis measures output conductance in volts of the device. The horizontal axis gives a measurement of elapsed time and the scale T indicates a time interval of sixty seconds.

The steady state is reached in about one minute. At the lower concentrations of urea the response is slightly slower and this can take up to three minutes depending upon the gelling conditions. The results obtained for the output response of conductance for similarly treated devices at various concentrations of urea in the range 0.1 to 100 mM are plotted in Figure 16.

In the graph of Figure 16, the vertical axis measures output conductance in millivolts of the device. The horizontal axis gives urea concentration in millimoles. The conductance output from the front side surface is shown by the dotted line, whilst that from the rear side surface is shown by the solid line.

Thus an overall enhancement of about twelve times in the strength of electrical signals as compared with that noted in the aforementioned patent application for a front side silicon device has been achieved with the present invention. This can make the device extremely sensitive for detecting very weak signals from other biological systems.

The reason for the fact that a substantial increase in the sensitivity of the biosensor device can be obtained is believed to be that a chelation or coordination bonding process occurs between the biological sample in the cell and the surface enlarged material of the support surface.

In an attempt to understand this point, a brief comparative scanning electron microscope examination of various silicon surfaces, such as front and rear side, p- and n-type, and metallised surfaces etched by different techniques, was carried out.

The semiconductor wafer material, as it is supplied by the manufacturer, has a polished finish on its front side and it is provided with 'gettering' material additions on its rear side. The purpose of these additions is to attract any impurities and defects which may be present on the front side of the wafer in the direction of the rear side. The front side can thereby be denuded of defects and dislocations which would be likely to interfere with the construction and operation of active devices which are intended to be fabricated thereon.

The gettering process operates to cause lattice damage and creates defects at the wafer rear side and these effects act as sinks for capturing impurities or defects from the wafer front side, during subsequent thermal processing.

In the comparative test, only the chemically wet-etched, metallised silicon surface appeared similar to the rear side gettered p-type silicon found most effective for the present invention. The n-type, unpolished, gettered silicon employed showed a very rough, uneven surface and this resulted in an electrical short-circuiting across the insulation material between the metal tracks. Based on electron micrographs, calculations were made to find the increase in surface area and surface on the rear side of the p-type silicon material, with the assumption that the etch pits present in the micrograph to be a right-angled circular cone shape. An increase of only about 1.5 times was found.

The defects such as interfacial misfit dislocations, grain boundaries and inpurities which are normally

present on the rear side of the silicon wafer are believed to act as 'active centres' or 'chelation sites' for the protein molecules and this can result in an improved wetting of the silicon substrate. The net result of the above two factors is entrapment of extra reaction product on the metal patterns which then results in an enhanced conductance signal. This effect could also be promoted by increasing the hydrophilic nature of the support surface by chemical means, for example by a silane treatment.

It is very possible that the newly available Enhanced Gettered (EG) polysilicon material and a soft rearside damaged silicon material may be advantageous to employ for the present invention. The EG silicon is produced by depositing pure, fine-grain polycrystalline silicon on a wafer rear side. This technique allows a maximum number of gettering sites to be applied to the rear surface of the wafer without creating excessive damage and a predisposition to slip. This can often be the case when an excessive degree of rear side damage has been applied. Surface modification could additionally be created by radiation bombardment, ion implantation, or other suitable means.

A fine grain polysilicon material of the kind used for the manufacture of solar cells is also considered to be a suitable surface material for the present invention.

The foregoing description of an embodiment of the invention has been given by way of example only, and a number of modifications may be made without departing from the scope of the invention as defined in the appended claims. For instance, instead of bonding the silicon chip to a twelve pin TO5 header package, the chip could be mounted on a different carrier such as a planar single layer leadless ceramic chip carrier (measuring 16.5 x 16.5 millimetres) having fortyfour gold plated nickel connection patterns on its surface and which was capable of supporting the sensor silicon chip on a central mounting pad. The external connections to the chip carrier, with its leadless gold plated edge connections which are joined internally to the surface connection patters, can then be made by enclosing the chip carrier in a special snap-on edge contact socket holder.

## Claims

1. A biosensor device comprising a reaction cell having electrical conductors separated by a support surface which is in contact with a biological sample when this is present in the cell, in which the support surface has a surface structure including an enlargement of the area of the surface material which can be brought into contact with said sample.

2. A device as claimed in Claim 1, in which the said support surface is a semiconductor material.

3. A device as claimed in Claim 2, in which the surface is of silicon.

4. A device as claimed in Claim 2 or 3, in which the said support surface comprises an electrically insulating film carried on the semiconductor substrate.

5. A biosensor device comprising an electrical insulation layer carried on a substrate of semiconductor material, the layer supporting a pair of substantially parallel thin film electrical conductors, electrical connection means for said conductors, a space between said conductors defining a reaction cell such that an electrical or thermal characteristic of a reaction component therein can be detected at said connection means, in which the semiconductor substrate material in the cell region has a surface structure including a roughly textured area where an enlargement of the substrate surface is present.

6. A method of constructing a biosensor device according to Claim 5, the method comprising the steps of taking a wafer of semiconductor material, selecting one major surface as a potential active wafer region, etching said surface so as to enlarge the surface area, oxidising said surface to form an electrical insulation film, depositing a pair of substantially parallel electrical conductors on said film and providing electrical connection means for the conductors.

7. A method of constructing a biosensor device according to Claim 5, the method comprising the steps of taking a wafer of semiconductor material, selecting one major surface as a potential active wafer region, oxidising said surface to form an electrical insulation film, depositing a metal layer on the oxide film, etching said metal layer so as to enlarge the surface area, depositing a pair of substantially parallel electrical conductors on said layer and providing electrical connection means for the conductors.

8. A method as claimed in Claim 6 or 7, in which the said major surface of the semiconductor material is a rough (unpolished) side of the wafer.

9. A method as claimed in Claim 6 or 7, in which the said etching step is effected by an electrochemical process.

10. A method of constructing a biosensor device, substantially as hereinbefore described with reference to any one of the accompanying drawings.

11. A biosensor device, substantially as hereinbefore described with reference to any one of the

accompanying drawings.

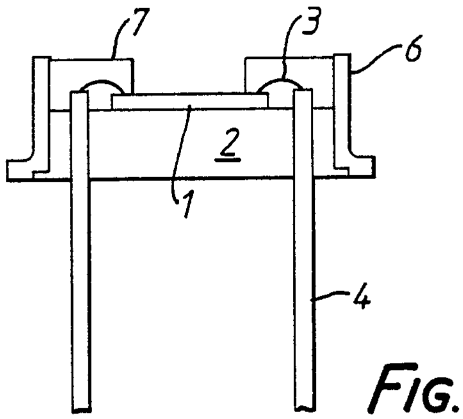


FIG.1.

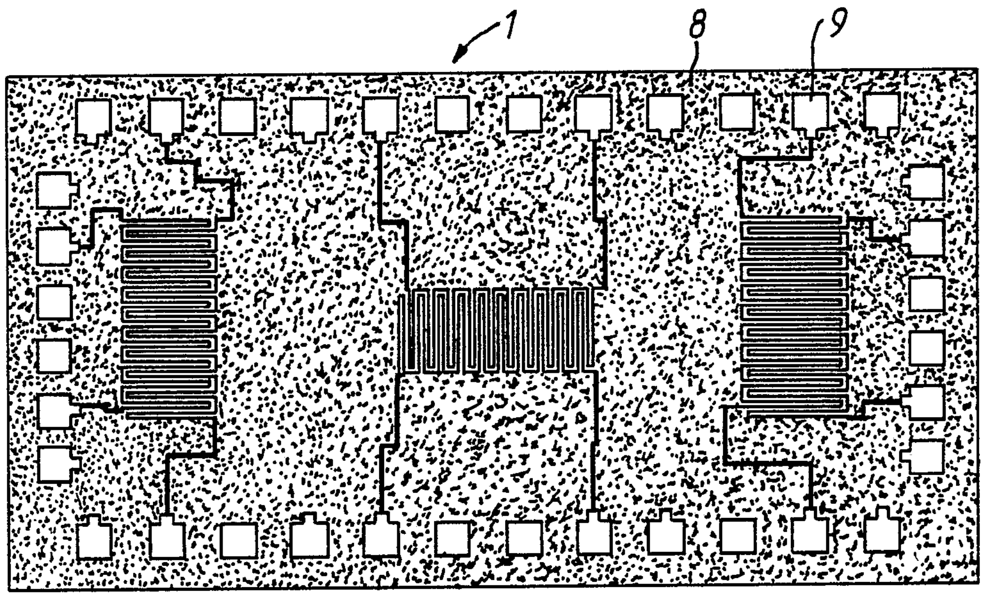


FIG.2.

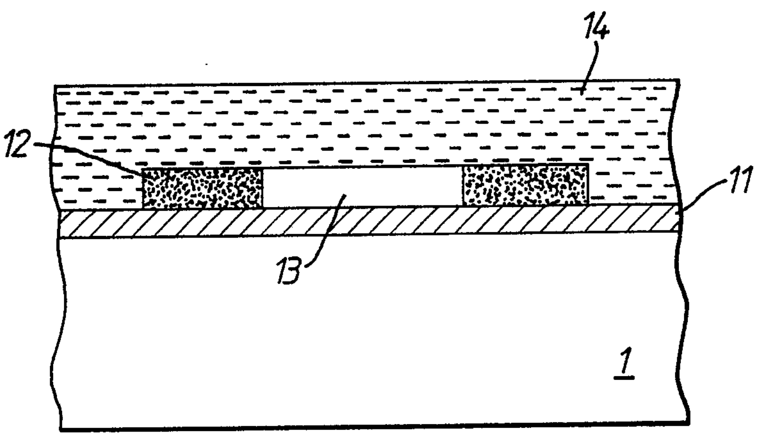


FIG.3.

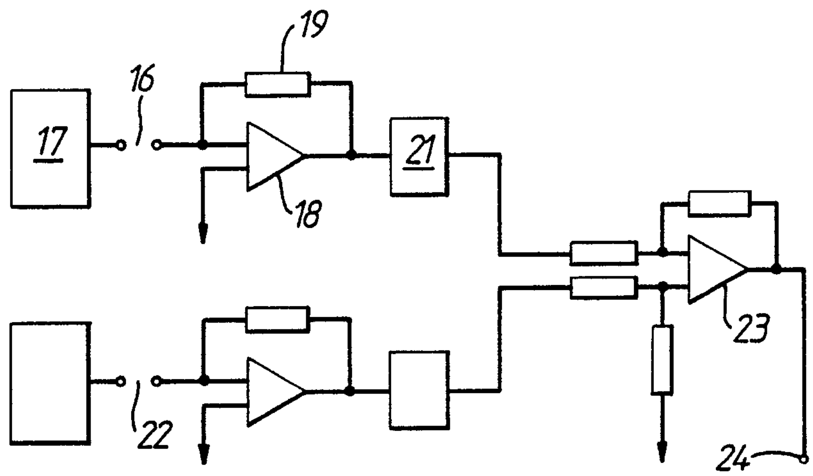


FIG. 4.

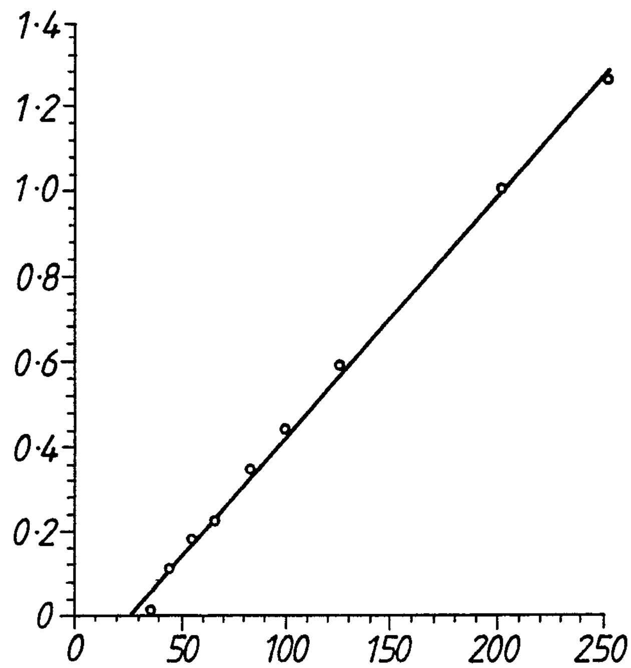


FIG. 5.

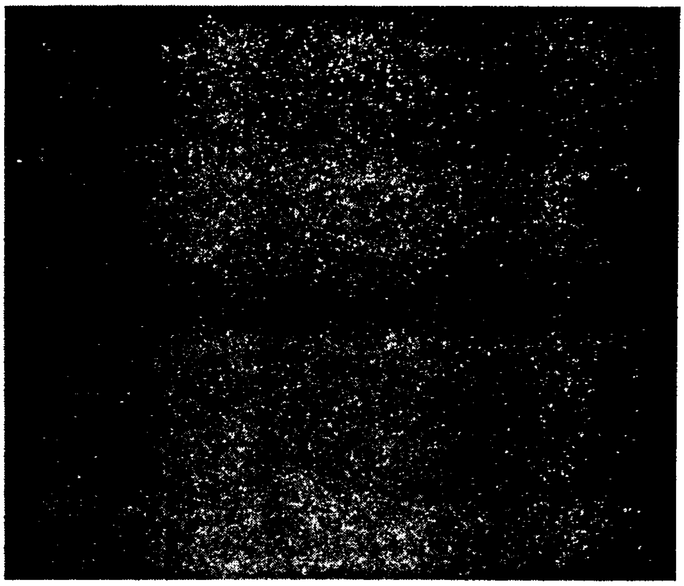

FIG. 6.

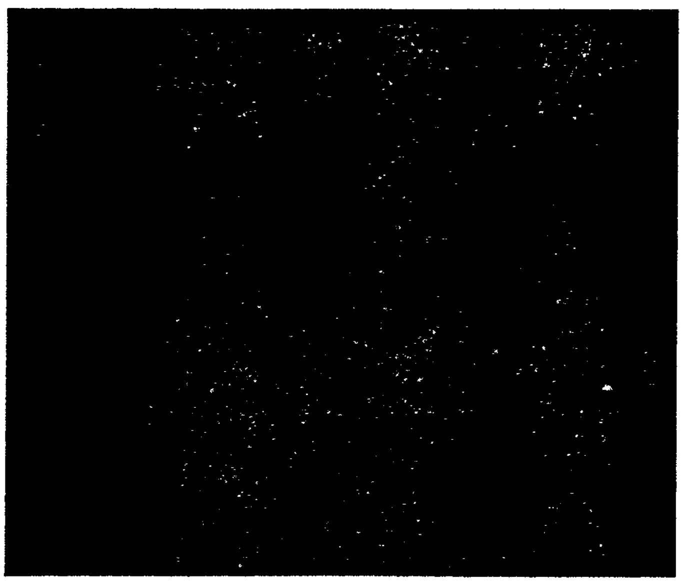

FIG. 7.

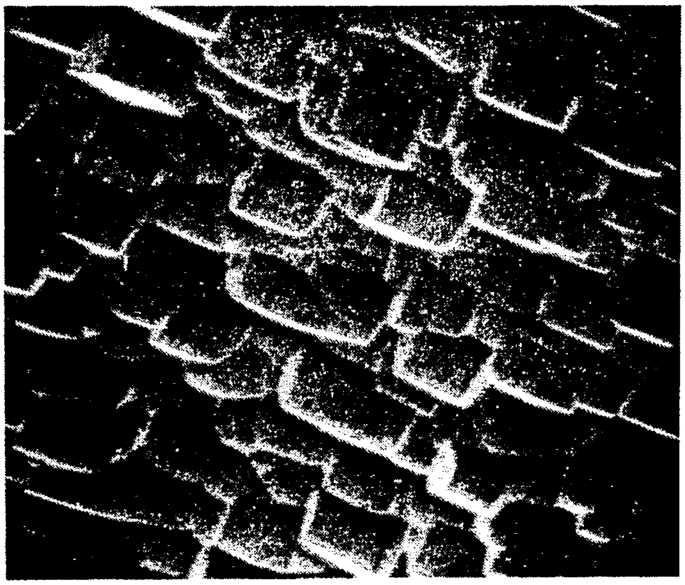

FIG. 8.

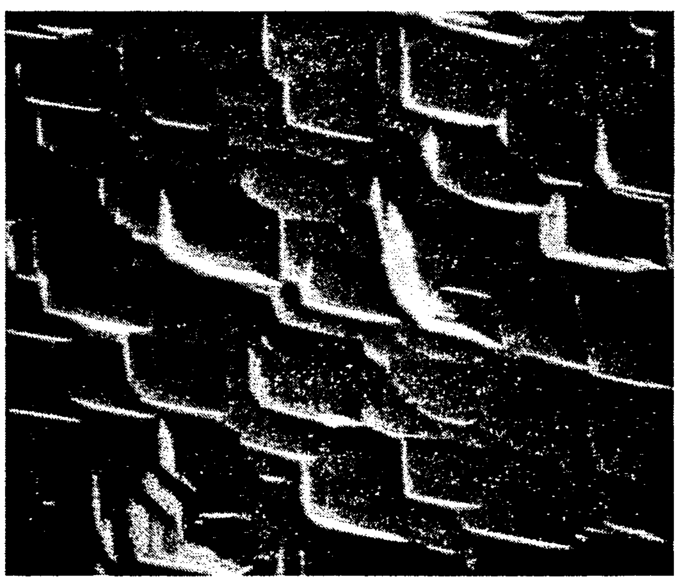

FIG. 9.

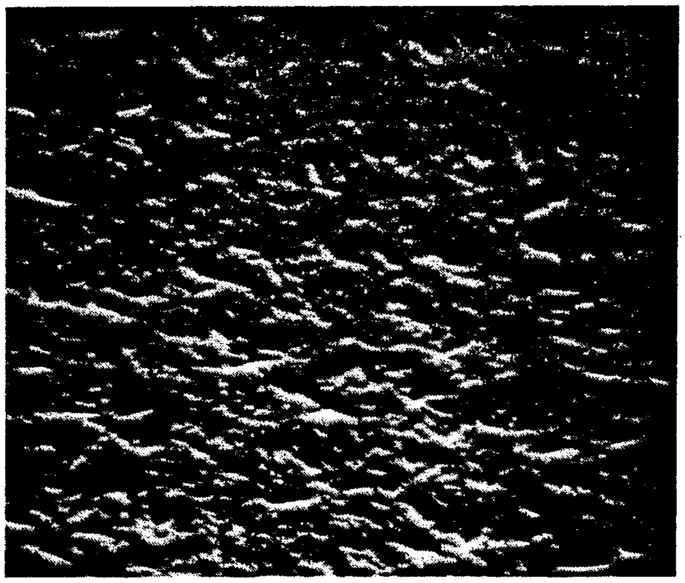

FIG.10.

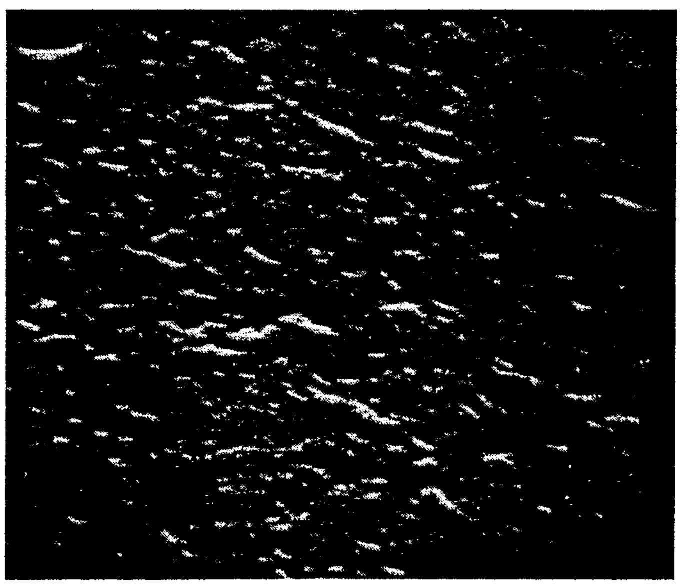

FIG.11.

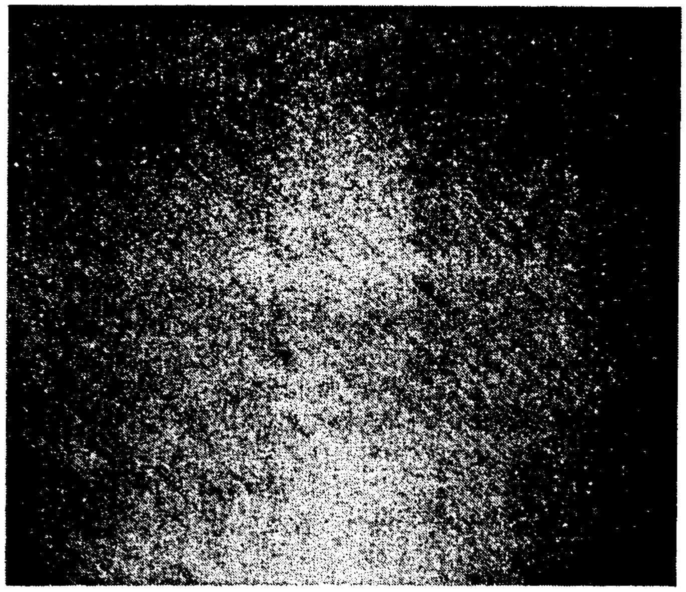

Fig. 12.

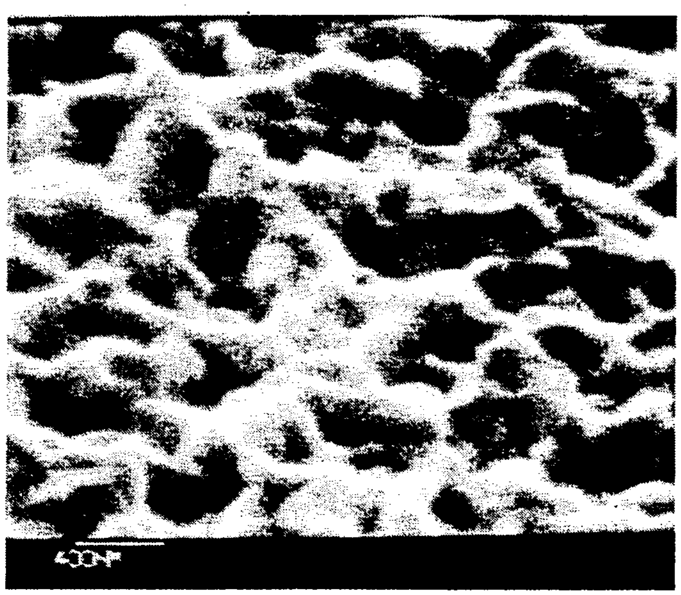

Fig. 13.

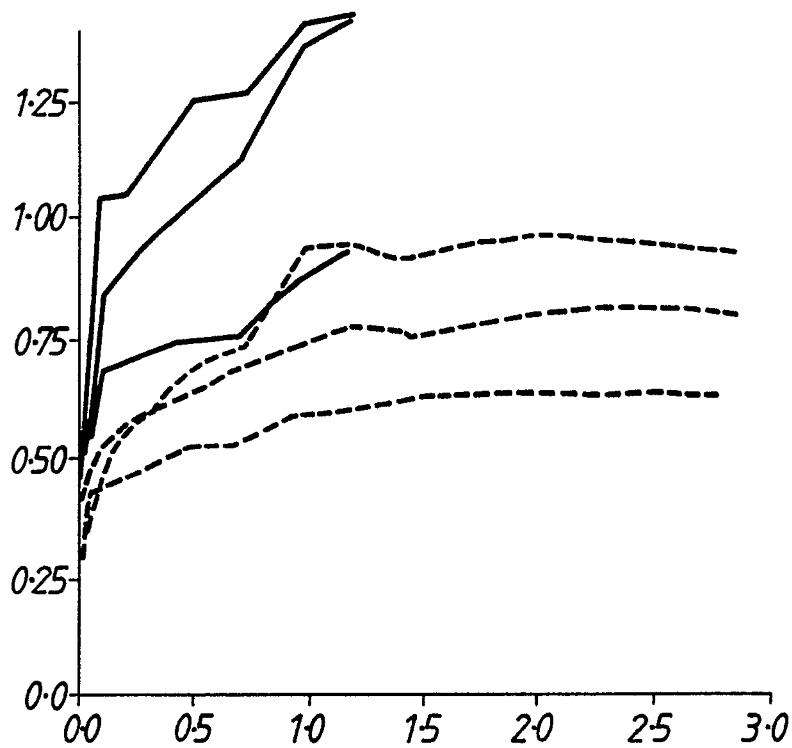


FIG. 14.

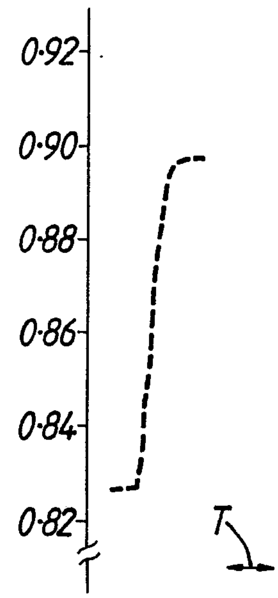


FIG. 15.

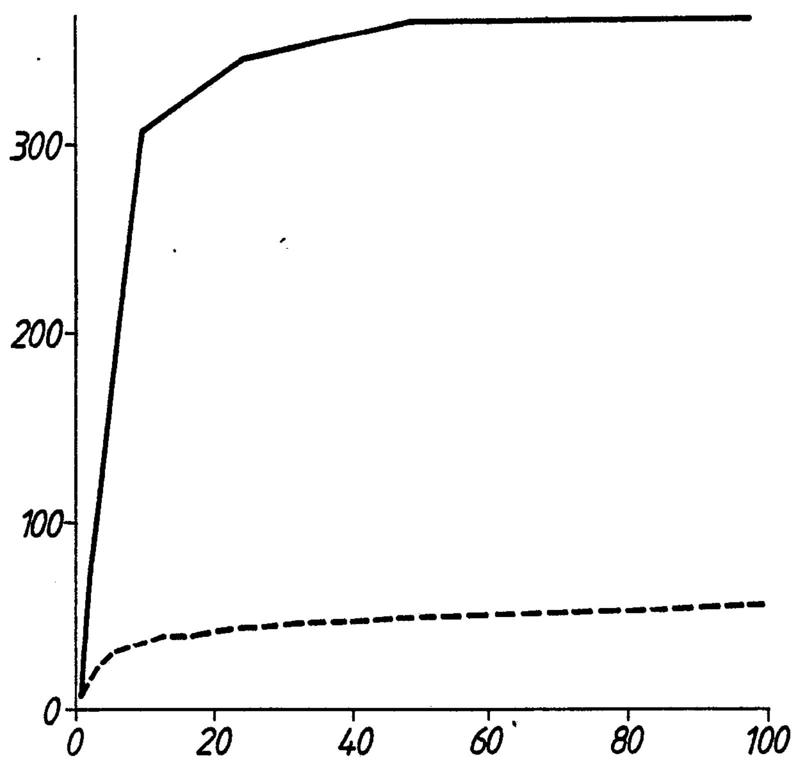


FIG. 16.

European Patent Office

# EUROPEAN SEARCH REPORT

Application Number

EP 89 31 0496

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3 634 132 (SHARP K.K.)<br>* Claims 1-5; page 3, lines 30-60; page 6, lines 5-42; figure 3 *<br>--- | 1-11 | C 12 M 1/40<br>G 01 N 27/12 |
| A | N.E.C. RESEARCH & DEVELOPMENT, no. 78, July 1985, pages 1-5, Tokyo, JP; T. KURIYAMA et al.: "A single chip biosensor"<br>* Pages 2-3: "Fabrication process" *<br>----- | 1-11 | |
| | | | **TECHNICAL FIELDS SEARCHED (Int. Cl.5)** |
| | | | C 12 M<br>G 01 N |

The present search report has been drawn up for all claims

| Place of search | Date of completion of the search | Examiner |
|---|---|---|
| THE HAGUE | 06-02-1990 | EPAILLARD P.J.H.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document